# DEMANDE DE BREVET EUROPEEN

(11) **EP 1 357 125 A2**
(43) Date de publication de la demande: **29.10.2003**
(21) Numéro de dépôt: 03017052.6
(22) Date de dépôt: 10.04.1998
(51) Int. Cl.: C07D 519/04

(54) **Procédé de préparation de dérivés halogénés d'alcaloides de vinca**

(30) Priorité: 10.04.1997 FR 9704398
(62) Demande divisionnaire de: 98920607.3
(71) Demandeur: PIERRE FABRE MEDICAMENT, 92100 Boulogne-Billancourt (FR)
(72) Inventeur: Duflos, Alain, 81290 Labruguiere (FR); Fahy, Jacques, 81290 Labruguiere (FR); Thillaye Du Boullay, Valérie, 81310 Lisle sur Tarn (FR); Barret, Jean-Marc, 81100 Castres (FR); Hill, Bridget, 81100 Castres (FR)
(74) Mandataire: Warcoin, Jacques

(57) **Abrégé**

Procédé de préparation de la 4'-déoxy-20',20'-difluorovinblastine ainsi qu'un procédé de préparation de la vinflunine à partir de la 4'-déoxy-20',20'-difluorovinblastine

## Description

Les alcaloïdes dimères du *Catharanthus roseus* et leurs dérivés, couramment appelés alcaloïdes de Vinca, sont largement utilisés en chimiothérapie anticancéreuse depuis une trentaine d'années. Ils sont représentés par quatre produits :
- deux composés naturels, la vinblastine et la vincristine,
- deux produits d'hémisynthèse, la vindésine obtenue à partir de la vinblastine, et la vinorelbine, synthétisée à partir des alcaloïdes monomères précurseurs, catharanthine et vindoline.

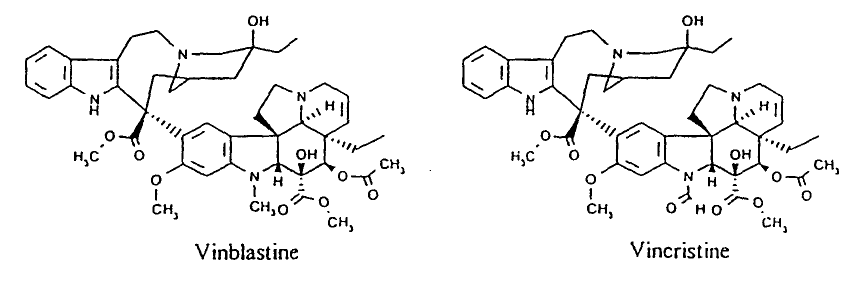

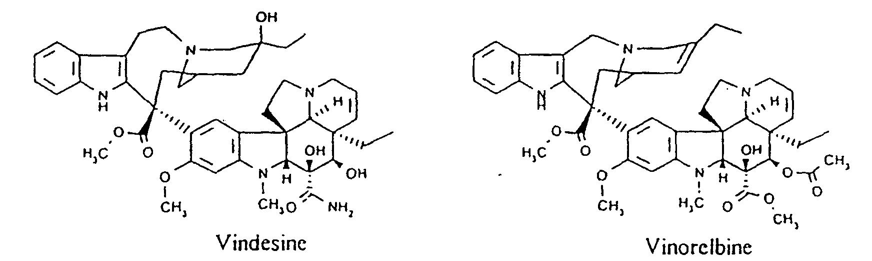

### Structure des alcaloïdes de Vinca utilisés en chimiothérapie

Dans le cadre de nos travaux de recherche visant à obtenir de nouveaux dérivés de cette famille pouvant conduire à de nouvelles applications en chimiothérapie anticancéreuse, nous avons adopté une démarche originale consistant à utiliser la réactivité exceptionnelle des milieux superacides, susceptible d'induire de profondes modifications dans ces molécules hautement fonctionnalisées.

Nous avons ainsi déjà préparé une série de composés difluorés en position 20', qui sont inaccessibles aujourd'hui par les méthodes classiques de synthèse (brevet FR 2 707 988 du 21.07.93, WO 95/03312), et dont les propriétés pharmacologiques sont particulièrement intéressantes.

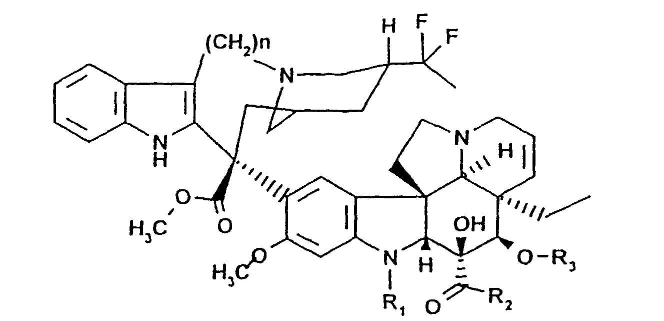

### Formule générale des composés décrits dans le brevet FR 2 707 988

L'application de cette chimie inhabituelle à ce type de molécules complexes nous a permis de préparer une nouvelle famille de composés halogénés, également inaccessibles par la chimie classique.

La présente invention, réalisée au Centre de Recherche Pierre Fabre, a pour objet de nouveaux dérivés d'alcaloïdes de Vinca, leur mode de préparation et leur application en thérapeutique. Les composés de l'invention possèdent la formule générale **1**, schématisée ci-dessous : 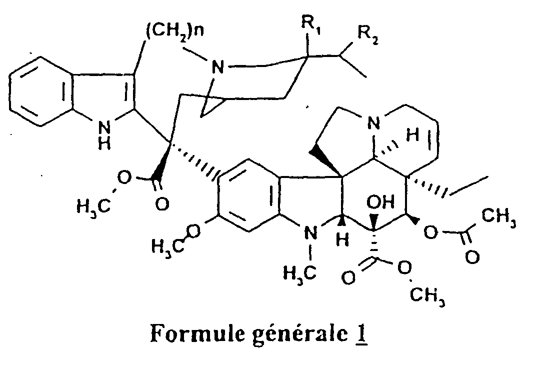 dans laquelle :
**n** = 1 ou 2,
**R**_{**1**} représente un atome d'hydrogène ou de fluor,
**R**_{**2**} représente un atome de chlore.

L'invention concerne également les sels des composés de formule générale **1** avec des acides minéraux ou organiques pharmaceutiquement acceptables. L'acide employé peut être, à titre d'exemple non limitatif, l'acide sulfurique ou l'acide tartrique.

L'invention concerne aussi bien les isomères correspondant aux configurations **R** et **S** des carbones 4' et 20' des composés de formule générale 1, que leurs mélanges en toute proportion.

Les dérivés de l'invention sont préparés par réaction d'un composé de formule générale **2** en milieu superacide, provenant du mélange d'un acide fort de Bronsted comme l'acide fluorhydrique, et d'un acide de Lewis fort comme le pentafluorure d'antimoine, en présence d'un réactif générateur d'espèces de type superélectrophile, suivant la dénomination proposée par G. Olah *(Ang. Chem. Int. Ed. Engl.,* 32, 767-88, 1993). Ce réactif peut être constitué d'un dérivé chloré comme le chlorure de méthylène, le chloroforme, le tétrachlorure de carbone, le 2,2-dichloropropane ou d'un mélange de ces dérivés en toutes proportions.

### La structure des composés de formule 2 est décrite ci-dessous :

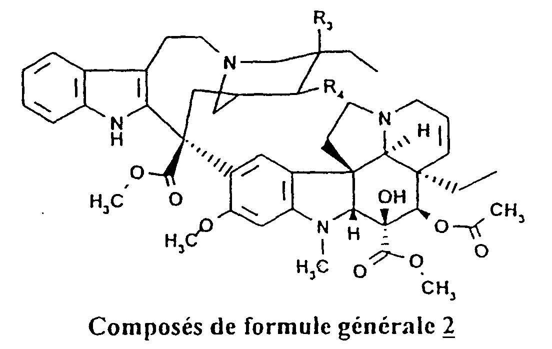

dans laquelle :
**R**_{**3**} représente un groupement hydroxyle et **R**_{**4**} un atome d'hydrogène, dans ce cas le composé **2** correspond à la vinblastine,
ou bien :
**R**_{**3**} et **R**_{**4**} formant une double liaison, dans ce cas le composé de formule **2** correspond à la 3',4'-anhydrovinblastine.

Les réactions en milieu superacide s'effectuent dans des récipients résistant à l'acide fluorhydrique comme le Téflon® ou un acier de composition adaptée. Le dérivé de formule **2**, dans laquelle **R**_{**3**} et **R**_{**4**} sont définis comme ci-dessus, est dissous dans l'acide fluorhydrique ou dans un dérivé chloré défini comme ci-dessus servant de solvant, et ajouté au mélange superacide, qui peut éventuellement déjà contenir une fraction de dérivé chloré. Cette addition est effectuée en maintenant la température du milieu à une valeur choisie, comprise entre -80 et -30 °C.

A basse température et en présence de chlorure de méthylène ou de tétrachlorure de carbone, les composés de formule **1** dans laquelle **n** = 2, **R**_{**1**} = H et **R**_{**2**} = Cl sont isolés de façon majoritaire. Les composés de formule **1** dans laquelle **n** = 2, **R**_{**1**} = F et **R**_{**2**} = Cl sont isolés du même milieu réactionnel en proportion plus faible. Ceci est résumé dans le schéma ci-dessous :

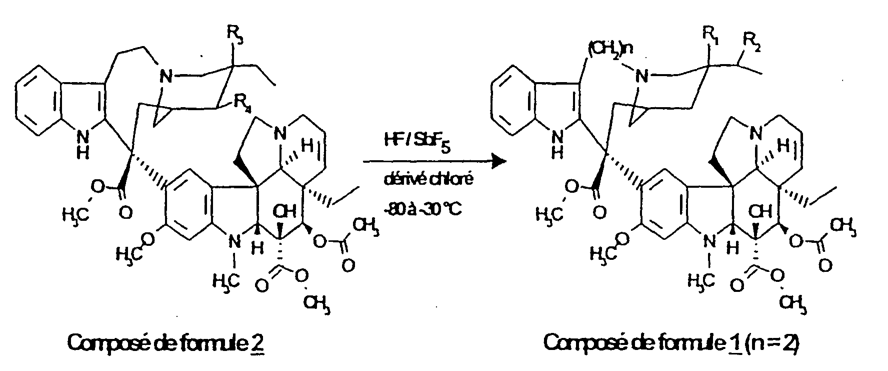

Les dérivés de formule générale **1**, dans lesquels **n** = 1, **R**_{**1**} et **R**_{**2**} étant définis comme ci-dessus, sont préparés par contraction du cycle C' (**n** = 2 → **n** = 1) des composés de formule générale **1** dans lesquels n = **2**, suivant les méthodes décrites dans la littérature (Eur. J. *Med.* Chem., **18**, 419-24, 1983), en particulier par action de la N-bromosuccinimide dans le chlorure de méthylène en présence d'un acide comme l'acide trifluoroacétique et à température inférieure à -10 °C. Après neutralisation en milieu basique, la 9'-bromoindolénine du dérivé de formule **1** dans laquelle **n** = 2, intermédiaire peu stable et non isolé, subit une hydrolyse de préférence dans un mélange [chlorure de méthylène : eau : tétrahydrofuranne], en présence ou non de tétrafluoroborate d'argent (AgBF₄), à une température comprise entre - 10° C et le reflux du solvant. L'addition d'AgBF₄; en quantité catalytique ou stoechiométrique par rapport au composé **1** ( **n** = 2), permet d'accélérer le cours de la réaction, si celle-ci n'est pas terminée après l'étape de neutralisation.

Le produit majoritaire de la réaction correspond au composé de formule **1** dans laquelle **n** = 1. Cette réaction est schématisée ci-dessous :

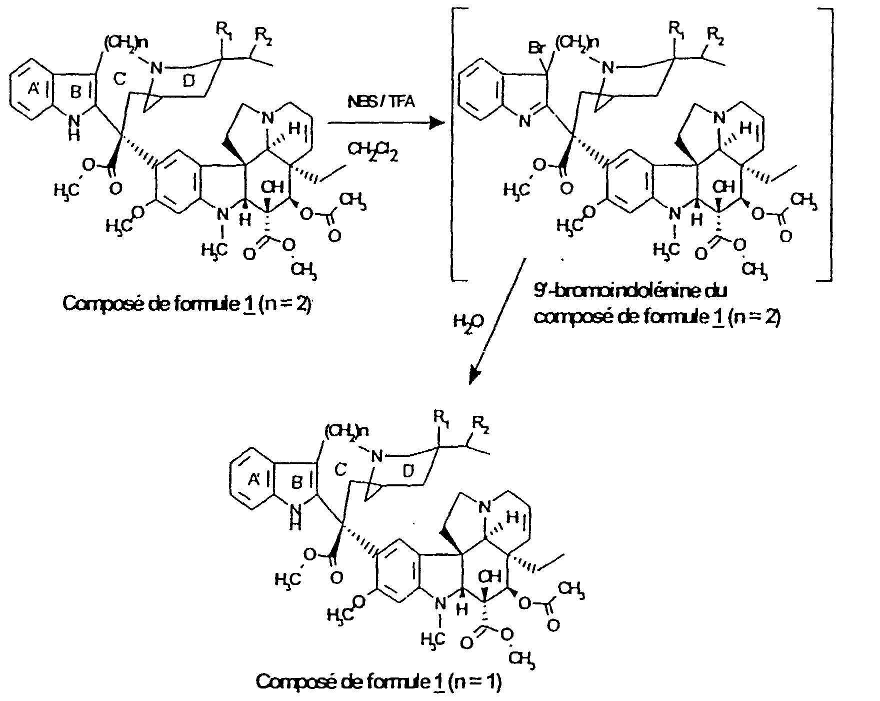

L'invention a également pour objet un nouveau procédé de synthèse de la 4'-déoxy-20',20'-difluorovinblastine, ainsi que de la 20',20'-difluoro-3',4'-dihydrovinorelbine ou vinflunine, composés revendiqués dans le brevet FR 2 707 988 du 21.07.93 (WO 95/03312).

En modifiant les conditions opératoires de la réaction en milieu superacide mettant en jeu les composés de formule **2** dans laquelle **n** = 2, **R**_{**3**} et **R**_{**4**} étant définis comme ci-dessus, on obtient préférentiellement la 4'-déoxy-20',20'-difluorovinblastine. Ces modifications de conditions opératoires consistent essentiellement à augmenter la température (entre -45 et -35 °C), à allonger le temps de réaction, ou à employer un superélectrophile de réactivité différente. En effet, nous avons montré dans une étude cinétique de la réaction que les composés isolables de formule **1** dans laquelle **n** = 2, **R**_{**1**} = H et **R**_{**2**} = Cl, majoritaires dans le cas précédent (41%) disparaissent au profit du produit difluoré en position 20' dans ces nouvelles conditions.

En optimisant les conditions opératoires, le rendement de cette réaction de difluoration est meilleur (50%) que celui obtenu par le procédé décrit dans le brevet cité ci-dessus, qui met notamment en jeu la N-bromosuccinimide (NBS) comme agent électrophile. En effet, le rendement indiqué pour la difluoration de la vinorelbine en présence de NBS n'est que de 25%.

Les composés de formule générale **2** sont la vinblastine quand **R**_{**3**} = OH et **R**_{**4**} = H, et la 3',4'-anhydrovinblastine quand **R**_{**3**} et **R**_{**4**} forment ensemble une double liaison. Le dérivé de formule **2** est dissous dans le dérivé chloré, de préférence le chloroforme ou le 2,2-dichloropropane, ou un mélange contenant du chloroforme ou du 2,2-dichloropropane, et ajouté au milieu superacide, en maintenant la température de ce dernier entre -45 et -35 °C. Le dérivé difluoré, 4'-déoxy-20',20'-difluorovinblastine, constitue alors le produit final majoritaire de la réaction. Le dosage par CLHP analytique indique une proportion de ce composé de l'ordre de 50%.

### Cette réaction est illustrée dans le schéma suivant :

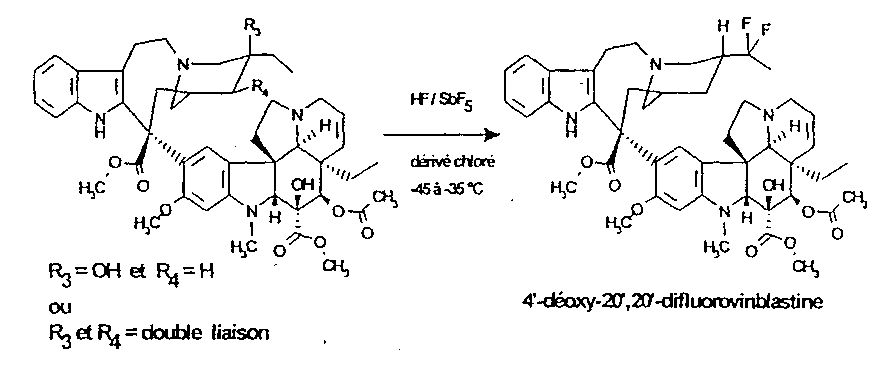

D'autres agents générateurs d'ions superélectrophiles comme le tétrabromure de carbone CBr₄, le dibromométhane CH₂Br₂ ou le tribromure de bore BBr₃ conduisent également à la 4'-déoxy-20',20'-difluorovinblastine de façon majoritaire.

Cette méthode constitue donc un nouveau procédé de préparation de la 4'-déoxy-20',20'-difluorovinblastine, avec un rendement supérieur à celui obtenu par la technique antérieure.

La 4'-déoxy-20',20'-difluorovinblastine obtenue suivant cette voie peut ensuite subir une contraction de son cycle C' (**n** = 2 → **n** = 1), comme décrit ci-dessus, par réaction avec la N-bromosuccinimide dans le chlorure de méthylène en présence d'un acide comme l'acide trifluoroacétique à température inférieure à -10 °C. Après neutralisation en milieu basique, le composé intermédiaire, 9'-bromoindolénine de la 4'-déoxy-20',20'-difluorovinblastine, peu stable et non isolé, subit une hydrolyse de préférence dans un mélange [chlorure de méthylène : eau : tétrahydrofuranne], en présence ou non de tétrafluoroborate. De la même façon que ci-dessus, l'addition d'AgBF₄ permet d'accroître la vitesse de la réaction.

Le composé majoritaire obtenu avec un rendement de l'ordre de 80% correspond à la 20',20'-difluoro-3',4'-dihydrovinorelbine ou vinflunine. Le schéma ci-dessous résume cette réaction :

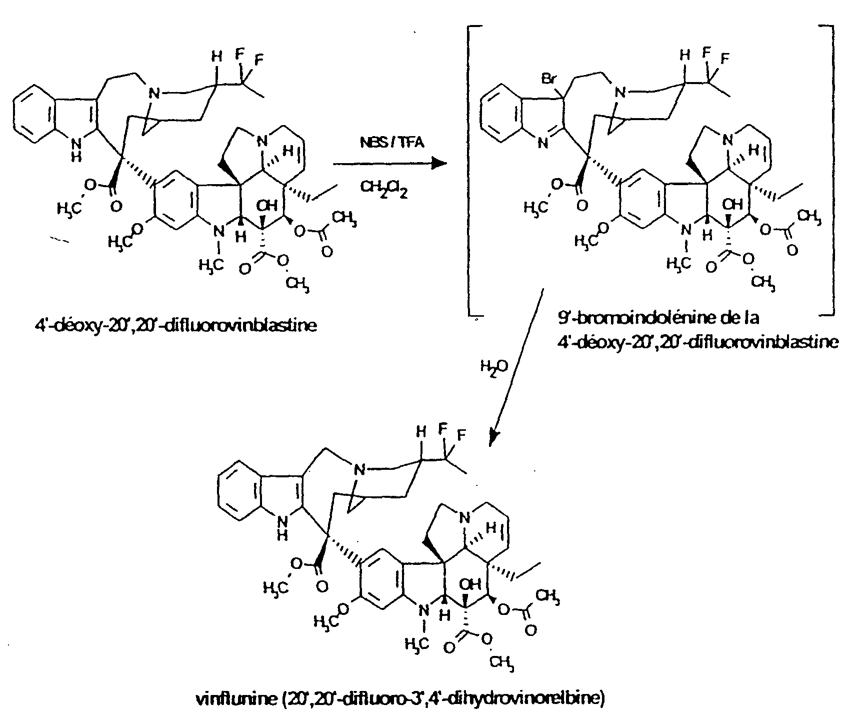

L'enchaînement de ces deux étapes constituent donc un nouveau procédé de synthèse de la vinflunine, plus avantageux que le précédent en ce sens que
1) la réaction de fluoration en milieu superacide est effectuée à partir de composés naturels comme la vinblastine ou la 3',4'-anhydrovinblastine, accessibles directement par extraction des feuilles de *Catharanthus roseus,* et non plus à partir de la vinorelbine, produit d'hémisynthèse plus onéreux,
2) le rendement de la réaction de difluoration par ce nouveau procédé est de l'ordre de 50% contre 25% avec le procédé précédent,
3) la purification par chromatographie des composés obtenus, notamment de la 4'-déoxy-20',20'-difluorovinblastine est moins délicate que dans le cas précédent.

Les exemples suivants illustrent l'invention, sans toutefois en limiter la portée. Les caractéristiques spectroscopiques confirment la structure des composés obtenus selon l'invention.

### Exemple 1 :

### 20'-chloro-4'-déoxyvinblastine 1. (n = 2, R₁ = H, R₂ = Cl)

Dans un récipient en Téflon de 250 ml, on prépare une solution de pentafluorure d'antimoine (60 g; 0,28 mole) dans 80 ml (4 moles) d'acide fluorhydrique anhydre que l'on refroidit à -60 °C. Sous agitation magnétique, on y ajoute successivement 1,63 ml (17 mmoles) de tétrachlorure de carbone, puis goutte-à-goutte une solution de 13,75 g (17 mmoles) de 3',4'-anhydrovinblastine 2 (**n** = 2, **R**_{**3**} et **R**_{**4**} formant une double liaison) dans 25 ml de chlorure de méthylène, en évitant que la température ne dépasse -40 °C. Après 30 minutes, le milieu réactionnel est versé avec grande précaution dans 1,5 litre d'une suspension aqueuse 3 M de Na₂CO₃ contenant 200 ml de chlorure de méthylène. Après décantation, la phase aqueuse est extraite par 100 ml de chlorure de méthylène. Les phases organiques sont regroupées, lavées par une solution saturée de NaCl, séchées sur MgSO₄ et évaporées.

L'analyse par CLHP analytique du résidu récupéré montre la présence de deux pics voisins intégrant au total pour 41% (21 et 20%) notés produit de l'**Exemple 1a** et produit de l'**Exemple 1b** dans les tableaux décrivant les spectres de RMN, correspondant aux deux diastéréoisomères en 20' de la 20'-chloro-4'-déoxyvinblastine.

La purification est effectuée par chromatographie sur colonne de silice, puis par CLHP préparative en phase inverse.

Les deux diastéréoisomères de la 20'-chloro-4'-déoxyvinblastine séparés sont salifiés sous forme de ditartrate par addition de deux équivalents (mole) d'acide tartrique dans l'eau, et lyophilisés.
C₄₆ H₅₇ Cl N₄ O₈, 2(C₄ H₆ O₆) : 1129,62
**Point de fusion :** > 260 °C

| | | | | | |
|---|---|---|---|---|---|
| **IR(KBr)** | 3448.17 | 1736.05 | 1615.36 | 1459.86 | 1372.51 |
| | 1231.89 | 1121.18 | 1067.54cm⁻¹. | | |

**Spectre de masse (D/CI NH**_{**3**}**)**:
MH + = 829.4

### Exemple 2 :

### 20'-chloro-4'-déoxyvinblastine 1. (n =2, R₁ = H, R₂ = Cl)

Ce composé est obtenu en suivant le mode opératoire décrit à l'**Exemple 1**, en remplaçant la 3',4'-anhydrovinblastine **2** (**n** = 2, **R**_{**3**} et **R**_{**4**} formant une double liaison) par la vinblastine **2** (**n** = 2, **R**_{**3**} = OH, **R**_{**4**} = H).

Les caractéristiques physico-chimiques et spectroscopiques des produits isolés sont identiques à celles des composés obtenus à l'**Exemple 1**.

### Exemple 3 :

### 20'-chloro-4'-déoxy-4'-fluorovinblastine 1. (n=2, R₁=F, R₂=Cl)

Ce composé est isolé du mélange réactionnel obtenu à l'**Exemple 1** ou à l'**Exemple 2**, à partir d'autres fractions moins polaires provenant de la chromatographie sur colonne de silice. La purification finale est effectuée par CLHP préparative en phase inverse.

La 20'-chloro-4'-déoxy-4'-fluorovinblastine est salifiée sous forme de ditartrate par addition de deux équivalents (mole) d'acide tartrique dans l'eau, et lyophilisée.
C₄₆ H₅₆ Cl F N₄ O₈ , 2(C₄ H₆ O₆) : 1147,61
**Point de fusion :** > 260 °C

| | | | | | |
|---|---|---|---|---|---|
| **IR(KBr)** | 3470.13 | 2951.45 | 1735.92 | 1616.62 | 1458.44 |
| | 1371.64 | 1228.52 | 1040.10 | 743.07 cm⁻¹. | |

**Spectre de masse (D/CI NH**_{**3**}**) :**
MH ⁺ = 847.4

### Exemple 4 :

### 20'-chloro-3',4'-dihydrovinorelbine 1. (n = 1, R₁ = H, R₂ = Cl)

On dissout 900 mg (1,08 mmoles) de 20'-chloro-4'-déoxyvinblastine **1** (**n** = 2, **R**_{**1**} = H, **R**_{**2**} = Cl), isolée suivant l'**Exemple 1**, dans 5 ml de chlorure de méthylène, additionné de 95 µl (1,2 mmoles) d'acide trifluoroacétique, refroidi à -45 °C. On place le montage à l'abri de la lumière, et on ajoute en solution dans 2 ml de CH₂Cl₂ et 95 µl (1,2 mmoles) de TFA 193 mg (1,08 mmoles) de N-bromosuccinimide, et on laisse agiter pendant 30 minutes. Le mélange est alors neutralisé par addition de 5 ml d'une solution saturée de NaHCO₃, puis aussitôt d'une solution de 211 mg (1,08 mmoles) de tétrafluoroborate d'argent dans un mélange de 5 ml de tétrahydrofuranne et 2 ml d'eau. On laisse revenir à température ambiante sous agitation magnétique pendant environ deux heures.

Après filtration, la phase organique est séparée, lavée par deux fois 10 ml d'eau, puis 10 ml d'une solution de NaCI saturé. Après séchage sur MgSO₄, la solution est évaporée et le résidu, est purifié par chromatographie sur colonne de silice, puis par CLHP préparative en phase inverse.
C₄₅ H₅₅ Cl N₄ O₈ : 815,41

| | | | | | |
|---|---|---|---|---|---|
| **IR (KBr)** | 3446 | 2950 | 1740.89 | 1459.16 | 1246.59 |
| | 1200.65 | 1042cm-¹. | | | |

**Spectre de masse (D/CI NH**_{**3**}**) :**
MH⁺= 815.4

### Exempte 5 :

### 20'-chloro-3',4'-dihydro-4'-fluorovinorelbine 1. (n = 1, R₁ = F, R₂ = Cl)

Ce composé est obtenu en suivant le mode opératoire décrit à l'**Exemple 4**, en remplaçant la 20'-chloro-4'-déoxyvinblastine par la 20'-chloro-4'-déoxy-4'-fluorovinblastine **1** (**n** = 2, **R**_{**1**} = F, **R**_{**2**} = Cl). La 20'-chloro-3',4'-dihydro-4'-fluorovinorelbine est purifiée par chromatographie dans des conditions identiques.
C₄₅ H₅₄ Cl F N₄ O₈ : 833,40

| | | | | | |
|---|---|---|---|---|---|
| **IR (KBr)** | 3446.83 | 2950.98 | 1742.73 | 1617.73 | 147.36 |
| | 1234.38 | 1042.23 | 996.50 | 742.15cm-¹. | |

**Spectre de masse (D/CI NH**_{**3**}**) :**
MH ⁺ = 833.5

Les déplacements chimiques des protons caractéristiques des structures nouvelles préparées ci-dessus sont rassemblés dans le tableau suivant :

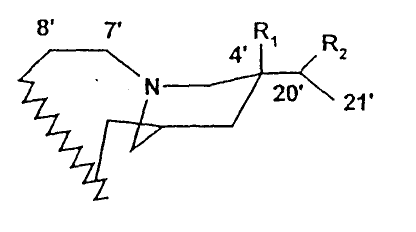

| Atome d'hydrogène | Produit de l'**Exemple** | | | |
|---|---|---|---|---|
| | **1a et 1b** | **3** | **4** | **5** |
| H4' | 1,95 (m, 1H) | - | 1,90 (m, 1H) | - |
| H₇' | 3,1-3,3 (m,2H) | 3,1 (m, 1H) | 4,32 (d, 1H) | 4,38 (d, 1H) |
| | | 3,3 (m, 1 H) | 4,46 (d, 1H) | 5,07 (d, 1H) |
| H_{8'} | 3,1-3,25 (m,2H) | 3,05 (m, 1H) | - | - |
| | | 3,45 (m,1H) | | |
| H₂₀' | 3,68 (m, 1 H) | 3,80 (m, 1 H) | 3,80 (m, 1 H) | 3,93 (m, 1H) |
| H_{21'} | 1,50 (d, 3H) | 1,44(d,3H) | 1,50 (d,3H) | 1,57 (d, 3H) |

La description des spectres de RMN ¹³C des produits nouveaux obtenus suivant les exemples ci-dessus est détaillée dans le tableau suivant :

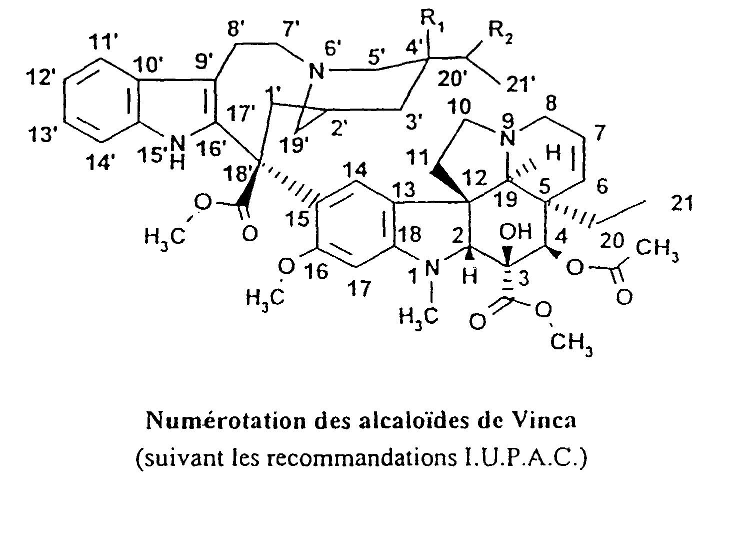

| **Caractéristiques de RMN 13**_{**C**} **des composés préparés** | | | | | |
|---|---|---|---|---|---|
| **Exemple** | **1a** | **1b** | **3** | **4** | **5** |
| Carbone n° | | | | | |
| 1 | N | N | N | N | N |
| 2 | 83.348 | 83.348 | 82.72 | 83.5 | 83.26 |
| 3 | 79.71 | 79.71 | 79.01 | 80.07 | 79.74 |
| 4 | 76.496 | 76.466 | 75.78 | 76.78 | 76.46 |
| 5 | 42.69 | 42.69 | 42.04 | 42.95 | 42.66 |
| 6 | 130.04 | 130.01 | 129.41 | 130.31 | 130.08 |
| 7 | 124.583 | 124.583 | 123.89 | 124.94 | 124.54 |
| 8 | 50.27 | 50.209 | 49.64 | 50.75 | 50.49 |
| 9 | N | N | N | N | N |
| 10 | 50.27 | 50.209 | 49.64 | 50.55 | 50.38 |
| 11 | 44.6 | 44.63 | 44.03 | 44.81 | 44.48 |
| 12 | 53.332 | 53.332 | 52.63 | 53.55 | 53.23 |
| 13 | 122.673 | 122.733 | 122.07 | 123 | 122.64 |
| 14 | 123.431 | 123.461 | 122.87 | 123.37 | 123.17 |
| 15 | 121.066 | 121.036 | 120.48 | 120.2 | 120.84 |
| 16 | 158.086 | 158.056 | 157.52 | 158.3) | 158.12 |
| 17 | 94.142 | 94.142 | 93.63 | 94.19 | 94.01 |
| 18 | 152.75 | 152.719 | 152.07 | 153.01 | 152.65 |
| 19 | 65.369 | 65.399 | 64.83 | 65.64 | 65.45 |
| 20 | 30.835 | 30.835 | 30.17 | 31 | 30.71 |
| 21 | 8.368 | 8.399 | 7.72 | 8.42 | 8.1 |
| 22 | 38.415 | 38.385 | 37.76 | 38.65 | 38.45 |
| 23 | 171.669 | 171.669 | 171.06 | 171.95 | 171.69 |
| 24 | 52.271 | 52.271 | 51.57 | 52.51 | 52.23 |
| 25 | 55.849 | 5.849 | 53:29 | 56.04 | 55.49 |
| 26 | 170.941 | 170.941 | 170.13 | 171.22 | 170.88 |
| 27 | 21.193 | 21.193 | 20.45 | 21.45 | 21.15 |
| 1' | 33.594 | 33.594 | 33 | 32.24 | 33.59 |
| 2' | 30.016 | 30.077 | 28.56 | 29.67 | 29.71 |
| 3' | 34.867 | 35.929 | 35.4 | 34.85 | 35.4 |
| 4' | 38.779 | 39.355 | 92.52 | 31 | 95.92 |
| 5' | 56.819 | 57.243 | 58.43 | 55 | 56.7 |
| 6' | N | N | N | N | N |
| 7' | 56.819 | 56.788 | 55.4 | 47.38 | 46.98 |
| 8 | 28.986 | 29.076 | 27.69 | Absent | Absent |
| 9' | 117.064 | 117.185 | 116.07 | 110.76 | 111.68 |
| 10' | 129.252 | 129.282 | 128.72 | 129.09 | 128.7 |
| 11' | 118.337 | 118.398 | 117.7 | 118.711 | 118.23 |
| 12' | 118.883 | 118.913 | 118.2 | 120.1 | 119.56 |
| 13' | 122.339 | 122.37 | 121.63 | 123.04 | 122.46 |
| 14' | 110.575 | 110.545 | 109.9 | 110.76 | 110.52 |
| 15' | 135.073 | 135.073 | 134.45 | 134.98 | 134.69 |
| 16' | N | N | N | N | N |
| 17' | 130.617 | 130.495 | 130.49 | 133.3 | 133.43 |
| 18' | 55.454 | 55.424 | 54.95 | 55.04 | 35.77 |
| 19' | 47.42 | 47.45 | 46.11 | 48.6 | 47.04 |
| 20' | 62.155 | 62.428 | 60.7 | 62.7 | 61.24 |
| 21' | 22.406 | 22.649 | 18.5 | 23.05 | 18.64 |
| 22' | 174.883 | 174.883 | 174.17 | 175 | 175 |
| 23' | 52.45 | 52.453 | 51.7 | 52.96 | 52.62 |

### Exemple 6 :

### 4'-déoxy-20',20'-difluorovinblastine.

Dans un réacteur en acier Asteloy de 6 litres, on prépare une solution de pentafluorure d'antimoine (750 g; 3,45 moles) dans 500 ml (25 moles) d'acide fluorhydrique anhydre que l'on refroidit à -45 °C. On y ajoute une solution de 107 g (0,12 mole) de dichlorhydrate de 3',4'-anhydrovinblastine dissous dans 250 ml de chloroforme en 30 minutes, en maintenant la température à -35 °C. On laisse sous agitation 30 minutes supplémentaires à cette température. On introduit ensuite 200 ml d'acétone en 30 minutes à -30 °C, puis 150 ml d'eau en 15 minutes à -25 °C. En laissant remonter la température à 20 °C, on ajoute 900 ml de chlorure de méthylène puis 650 ml d'eau. Après décantation, la phase aqueuse est extraite par 100 ml de chlorure de méthylène. Les phases. organiques sont regroupées, et neutralisées à température inférieure à 20 °C par 600 ml de KOH à 10 %. Après décantation, la phase organique est agitée pendant une nuit en présence de 800 ml d'ammoniaque à 10% avant d'être lavée deux fois par 400 ml d'eau, séchée sur MgSO₄ puis évaporée.

La purification est effectuée par chromatographie sur colonne de silice éluée par un mélange toluène : acétone (65 : 35). Le résidu obtenu après évaporation est dissous dans 80 ml de chlorure de méthylène et précipité par addition de 350 ml d'éther isopropylique. Après filtration, on récupère 41 g (40%) de 4'-déoxy-20',20'-difluorovinblastine.

Les caractéristiques physico-chimiques et spectroscopiques du composé isolé sont identiques à celles du produit obtenu suivant le procédé décrit dans le brevet FR 2 707 988 du 21.07.93 (WO 95/03312).

### Exemple 7 :

### 4'-déoxy-20',20'-difluorovinblastine.

Dans un récipient en Téflon de 125 ml, on prépare une solution de pentafluorure d'antimoine (8,6 g; 40 mmoles) dans 7,2 ml (360 mmoles) d'acide fluorhydrique anhydre que l'on refroidit à -45 °C. Sous agitation magnétique, on y ajoute successivement 0,94 ml (9 mmoles) de 2,2-dichloropropane, puis goutte-à-goutte une solution de 0,71 g (0,9 mmoles) de 3',4'-anhydrovinblastine **2** (**n** = 2, **R**_{**3**} et **R**_{**4**} formant une double liaison) dans 3,6 ml d'acide fluorhydrique, en évitant que la température ne dépasse -35 °C. Après 25 minutes, le milieu réactionnel est versé avec grande précaution dans 300 ml d'une solution aqueuse 3 M de Na₂CO₃. Après décantation, la phase aqueuse est extraite par 50 ml de chlorure de méthylène. Les phases organiques sont regroupées, lavées par une solution saturée de NaCl, séchées sur MgSO₄ et évaporées.

Le résidu contient 46% de 4'-déoxy-20',20'-difluorovinblastine, identique au produit obtenu suivant le procédé décrit dans le brevet FR 2 707 988 du 21.07.93 (WO 95/03312).

### Exemple 8 :

### 4'-déoxy-20',20'-difluorovinblastine

Ce composé est obtenu en suivant le mode opératoire décrit à l'**Exemple 7**, en remplaçant le 2,2-dichloropropane par le tétrabromure de carbone CBr₄ lors du traitement en milieu superacide.

Les caractéristiques physico-chimiques et spectroscopiques du composé isolé sont identiques à celles du produit obtenu suivant le procédé décrit dans le brevet FR 2 707 988 du 21.07.93 (WO 95/03312).

### Exemple 9 :

### 4'-déoxy-20',20'-difluorovinblastine

Ce composé est obtenu en suivant le mode opératoire décrit à **l'Exemple 7,** en remplaçant le 2,2-dichloropropane par le tribromure de bore BBr₃ lors du traitement en milieu superacide.

Les caractéristiques physico-chimiques et spectroscopiques du composé isolé sont identiques à celles du produit obtenu suivant le procédé décrit dans le brevet FR 2 707 988 du 21.07.93 (WO 95/03312).

### Exemple 10 :

### 4'-déoxy-20',20'-difluorovinblastine

Ce composé est obtenu en suivant le mode opératoire décrit à l'**Exemple 7**, en remplaçant le 2,2-dichloropropane par le dibromométhane CH₂Br₂ lors du traitement en milieu superacide.

Les caractéristiques physico-chimiques et spectroscopiques du composé isolé sont identiques à celles du produit obtenu suivant le procédé décrit dans le brevet FR 2 707 988 du 21.07.93 (WO 95/03312).

### Exemple 11:

### 20',20'-difluoro-3',4'-dihydrovinorelbine (vinflunine).

On dissout 90 g (0,108 mole) de 4'-déoxy-20',20'-difluorovinblastine dans 800 ml de chlorure de méthylène qu'on refroidit à -45 °C, et auxquels on ajoute 10,4 ml (0,135 mole) d'acide trifluoroacétique. On place le montage à l'abri de la lumière, et on y verse durant environ 30 minutes une solution composée de 19,3 g (0,108 mole) de N-bromosuccinimide dans un mélange de 200 ml de chlorure de méthylène et 10,4 ml (0,135 mole) d'acide trifluoroacétique, en veillant à ce que la température du milieu ne remonte pas au-dessus de -45 °C.

Après 30 minutes, on neutralise le mélange par addition de 300 ml d'une solution à 10% de NaHCO₃, puis aussitôt une solution de 23,4 g (0,12 mole) de tétrafluoroborate d'argent dans un mélange de 300 ml de tétrahydrofurane et 100 ml d'eau. On laisse revenir à température ambiante sous agitation pendant environ deux heures.

Après filtration, la phase organique est séparée, lavée par deux fois 150 ml d'eau. Après séchage sur MgSO₄, la solution est évaporée et le résidu est dissous dans 500 ml de méthanol puis évaporé pour obtenir 97 g de résidu sec, contenant 88% de 20',20'-difluoro-3',4'-dihydrovinorelbine (vinflunine).

La purification est effectuée par CLHP préparative sur silice greffée C₁₈ (granulométrie 15-25 µ), avec un éluant composé d'eau, d'acide acétique, d'acétate d'ammonium, de méthanol et d'acétonitrile. Les fractions purifiées sont concentrées à moitié, amenées à pH = 7 et extraites deux fois par du toluène. La phase organique est lavée trois fois par de l'eau distillée, puis dosée par HPLC analytique.

La vinflunine, en solution dans le toluène, est extraite par une solution aqueuse contenant exactement deux équivalents (mole) d'acide tartrique. La phase aqueuse est ensuite lyophilisée pour obtenir le ditartrate de vinflunine.

Les caractéristiques physico-chimiques et spectroscopiques du composé isolé sont identiques à celles du produit obtenu suivant le procédé décrit dans le brevet FR 2 707 988 du 21.07.93 (WO 95/03312).

Comme les alcaloïdes antitumoraux du *Catharanthus roseus,* les composés préparés suivant l'invention sont des "poisons du fuseau mitotique".

Cette propriété a été confirmée par la mesure de l'inhibition de la polymérisation de la tubuline en microtubules en présence des composés de l'invention, en suivant la méthode décrite par R. C. Weisenberg *(Science* **177**, 1196-7, 1972). Les résultats sont exprimés en IC₅₀, qui correspond à la concentration de composé qui provoque 50 % d'inhibition de la polymérisation. Ce phénomène est aisément suivi et quantifié par l'intermédiaire des-variations de la densité optique.

A titre d'exemple, le tableau ci-dessous montre les résultats obtenus avec deux dérivés préparés suivant l'invention :

| **Produit** | **IC**_{**50**} **(µM)** |
|---|---|
| **Exemple 1** | 1.99 |
| **Exemple 3** | 1.54 |
| **Vinorelbine** | 1.70 |

Compte-tenu de cette propriété pharmacologique caractéristique des alcaloïdes de Vinca, les composés de la présente invention peuvent être utilisés en chimiothérapie anticancéreuse.

Les préparations pharmaceutiques contenant ces principes actifs peuvent être mises en forme pour l'administration par voie orale, intraveineuse ou sous-cutanée, de manière classique, bien connue de l'homme du métier.

## Revendications

1. Procédé de préparation de la 4'-déoxy-20',20'-difluorovinblastine, **caractérisé en ce que** l'on fait réagir un composé de formule générale 2 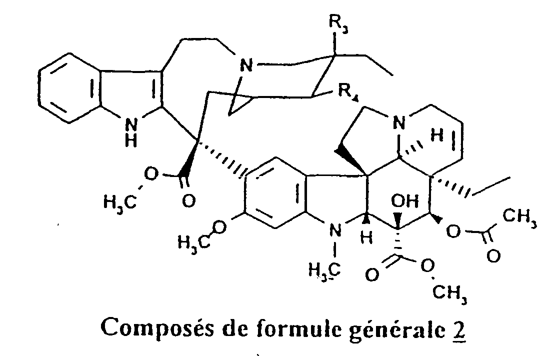 dans laquelle :
R₃ représente un groupement hydroxyle et R₄ un atome d'hydrogène,
ou bien :
R₃ et R₄ formant une double liaison,
en milieu superacide, provenant de l'association d'un acide de Bronsted comme l'acide fluorhydrique HF et d'un acide de Lewis comme le pentafluorure d'antimoine SbF₅, en présence d'un réactif générateur d'espèces de type superélectrophile, la température du milieu réactionnel étant comprise entre -45 et -35°C.

2. Procédé de préparation du composé selon la revendication 1, **caractérisé en ce que** le réactif générateur d'espèces de type superélectrophile est un dérivé chloré générateur d'ions superélectrophiles choisi parmi le chloroforme, le 2,2-dichloropropane et un mélange de ces derniers.

3. Procédé de préparation du composé selon la revendication 1, **caractérisé en ce que** le réactif générateur d'espèces de type superélectrophile est choisi parmi le tétrabromure de carbone CBr₄, le dibromométhane CH₂Br₂ et la tribromure de bore BBr3.

4. Procédé de préparation de la vinflunine, **caractérisé en ce que** l'on fait réagir la 4'-déoxy-20',20'-difluorovinblastine obtenue selon l'une quelconque des revendications 1 à 3 avec un agent halogénant comme la N-bromosuccinimide, dans un solvant chloré comme le chlorure de méthylène et en présence d'un acide comme l'acide trifluoroacétique à une température inférieure à -10°C, conduisant à la 9'-bromoindolénine de la 4'-déoxy-20',20'-difluorovinblastine correspondante, intermédiaire non isolé, de sorte à obtenir la 20',20'-difluoro-3',4'-dihydrovinorelbine ou vinflunine.

5. Procédé selon la revendication 4, **caractérisé en ce que** l'on neutralise le mélange réactionnel contenant la 9'-bromoindolénine de la 4'-déoxy-20',20'-difluorovinblastine par un milieu basique, suivi d'une hydrolyse de préférence par un mélange [chlorure de méthylène : eau : tétrahydrofuranne], en présence ou non de tétrafluoroborate d'argent à une température comprise entre -10 °C et le reflux du solvant, selon le schéma suivant :
